# EUROPÄISCHE PATENTSCHRIFT

(11) **EP 1 537 789 B1**
(45) Veröffentlichungstag und Bekanntmachung des Hinweises auf die Patenterteilung: **27.09.2006**
(21) Anmeldenummer: 03027832.9
(22) Anmeldetag: 04.12.2003
(51) Int. Cl.: A23L 1/30, A61K 36/00, A61K 36/45, A61K 8/97

(54) **Zubereitung für die orale und/oder topische Anwendung (I)**
Composition for oral or cosmetic application
Composition pour application orale ou cosmétique

(43) Veröffentlichungstag der Anmeldung: 08.06.2005
(73) Patentinhaber: Cognis IP Management GmbH, 40589 Düsseldorf (DE); Cognis France, S.A.S., 31360 Boussens (FR)
(72) Erfinder: Buchwald-Werner, Sybille, Dr., 40589 Düsseldorf (DE); Le Hen Ferrenbach, Catherine, 77100 Meaux (FR); Carite, Christophe, 87570 Rilhac-Rancon (FR); Blasquez, José Fernandez, 08228 Terrassa (ES); Rull Prous, Santiago, 08034 Barcelona (ES)

(56) Entgegenhaltungen:
- EP-A- 1 314 420
- EP-A- 1 344 516
- WO-A-02/100329
- DE-U- 20 115 071
- PATENT ABSTRACTS OF JAPAN vol. 2003, no. 03, 5. Mai 2003 (2003-05-05) & JP 2002 332224 A (KOSE CORP;TOKIWA SHOKUBUTSU KAGAKU KENKYUSHO:KK), 22. November 2002 (2002-11-22)
- CAI Q, RAHN RO, ZHANG R: "Dietary flavonoids, quercetin, luteolin and genistein, reduce oxidative DNA damage and lipid peroxidation and quench free radicals." CANCER LETTERS, Bd. 119, 1997, Seiten 99-107, XP002275780

## Beschreibung

### Gebiet der Erfindung

Die Erfindung befindet sich sowohl auf dem Gebiet der Nahrungsmittelzusatz- bzw. Nahrungsmittelergänzungsstoffe als auch auf dem Gebiet der Sonnenkosmetik und betrifft neue Zubereitungen zur oralen bzw. topischen Anwendung, enthaltend spezielle Wirkstoffmischungen, die den Körper vor den schädlichen Auswirkungen übermäßiger Sonneneinstrahlung schützen.

### Stand der Technik

In den letzten Jahren hat der Markt für Nahrungsmittelzusatzstoffe einen ungeheuren Aufschwung erfahren. Vom Verbraucher werden sowohl Produkte gewünscht, die in einem eher undifferenzierten Ansatz den körperlichen Wohlbefinden nutzen und die Abwehrkräfte steigern, wie dies beispielsweise typisch für Vitamine ist, als auch solche, die unter den Begriffen "Health Food" oder "Dietary Supplements" bekannt sind und beispielsweise den Fettabbau oder den Muskelaufbau beschleunigen. So wird beispielsweise in der internationalen Patentanmeldung **WO 97/46230** (WARF) vorgeschlagen, konjugierte Linolsäure für diesen Zweck einzusetzen. Ein weiteres Beispiel für den wachsenden Markt für Nahrungsmittelergänzungsstoffe kann unter der Überschrift "Cosmetic inside" oder "Beauty inside" zusammengefasst werden. Hier geht es darum, Haut und Haare sowie Fingernägel in Ihrer physiologischen Funktion zu unterstützen und Erscheinungen wie z.B. Hautalterung zu verlangsamen. Lange bekannt für solche Anwendungen sind z.B. Carotinoide für den Sonnenschutz. Die Verwendung von Carotinoiden und/oder Pflanzenextrakten zum Schutz vor dem Altern wird zum Beispiel von EP 1 314 420; JP 2003/332 24; und WO 02/100 329 offenbart.

In diesem Zusammenhang besteht jedoch das Bedürfnis, Zubereitungen zur Verfügung zu stellen, die einerseits die einzelnen Aufgabenstellungen im Vergleich zum Stand der Technik in verbesserter Weise lösen - also beispielsweise die gleichen Effekte bei geringerer Dosierung ergeben - als auch unterschiedliche Aufgabenstellungen miteinander verbinden.

Die Aufgabe der vorliegenden Erfindung hat darin bestanden neue Zubereitungen zur Verfügung zu stellen, die sowohl bei oraler Verabreichung im Sinne von Nahrungsmittelergänzungsstoffen (z.B. "Functional Food") als auch bei topischer Anwendung im Sinne eines Sonnenschutzmittels den Körper vor den negativen Auswirkungen übermäßiger Sonneneinstrahlung, also UVA- und UVB-Exposition schützen, die kosmetische Beschaffenheit von Haut und Haaren verbessern und gleichzeitig über entzündungshemmende Eigenschaften verfügen.

### Beschreibung der Erfindung

Gegenstand der Erfindung sind Zubereitungen für die orale und/oder topische Anwendung, enthaltend
(a) Carotinverbindungen,
(b) Tocopherole und
(c) Extrakte der Pflanze *Passiflora incarnata* bzw. deren aktive Wirkstoffe sowie gegebenenfalls
(d) Extrakte der Pflanze *Vaccinium myrtillus* bzw. deren aktive Wirkstoffe.

Überraschenderweise wurde gefunden, dass sich die ternären bzw. quaternären Gemische durch eine synergistische Wirkung im Hinblick auf antioxidative und entzündungshemmende Eigenschaften sowie den Schutz der Augen vor UV-Strahlung auszeichnen. Unter Verwendung der erfindungsgemäßen Wirkstoffgemische lassen sich sowohl Sonnenschutzmittel für die topische Anwendung herstellen als auch - beispielsweise in Kapseln eingeschlossen - Nahrungsmittelergänzungsstoffe, die bei oraler Verabreichung den Körper vor negativen Auswirkungen übermäßiger Sonneneinstrahlung schützen.

### Carotinverbindungen

Unter Carotinverbindungen, die die Komponente (a) bilden, sind im wesentlichen Carotine und Carotinoide zu verstehen. Carotine stellen eine Gruppe von 11- bis 12fach ungesättigten Triterpenen dar. Von besonderer Bedeutung sind die drei isomeren α-, β- und γ-Carotine, die alle über das gleiche Grundgerüst mit 9 konjugierten Doppelbindungen, 8 Methylverzweigungen (einschließlich möglicher Ringstrukturen) und einer β-Ionon-Ringstruktur an einem Molekülende verfügen und ursprünglich als einheitlicher Naturstoff angesehen worden waren. Nachstehend sind eine Reihe von Carotinverbindungen abgebildet, die als Komponente (b) in Frage kommen, ohne dass es sich um eine abschließende Aufzählung handelt.

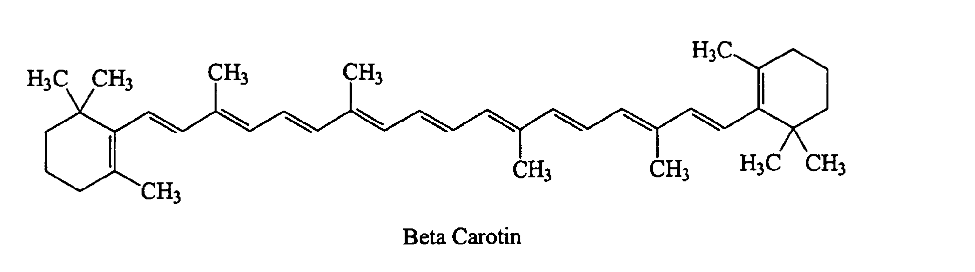

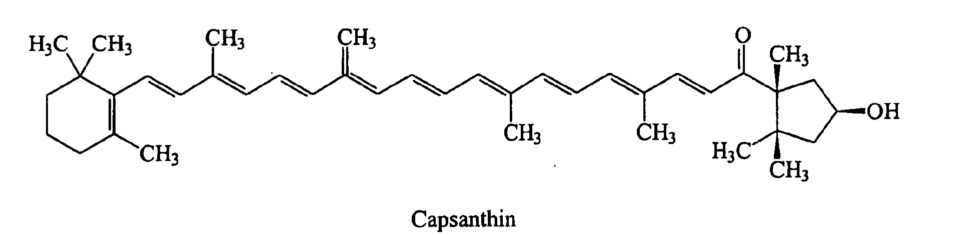

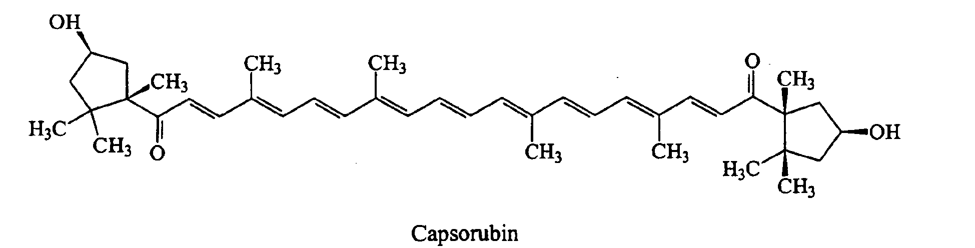

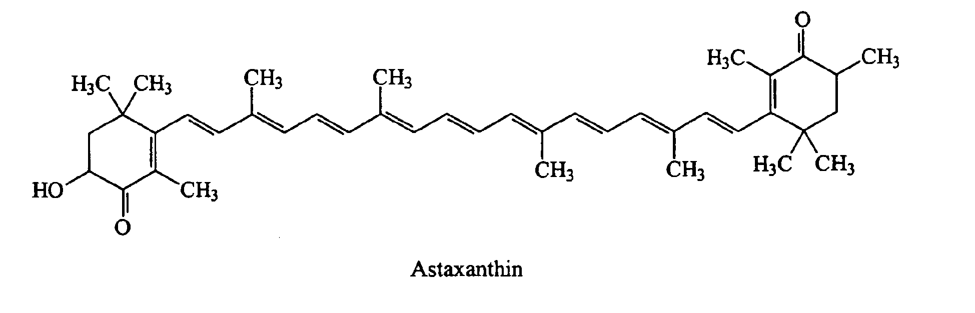

Neben den bereits genannten Isomeren kommen auch das δ-, ε- und ζ-Carotin (Lycopin) in Betracht, wobei freilich das β-Carotin (Provitamin A) wegen seiner hohen Verbreitung von besonderer Bedeutung ist; im Organismus wird es enzymatisch in zwei Moleküle Retinal gespalten. Unter Carotinoiden versteht man sauerstoffhaltige Derivate der Carotine, die auch als Xanthophylle bezeichnet werden, und deren Grundgerüst aus 8 Isopreneinheiten (Tetraterpene) bestehen. Man kann sich die Carotinoide aus zwei C₂₀-Isoprenopiden derart zusammengesetzt denken, dass die beiden mittleren Methylgruppen in 1,6-Stellung zueinander stehen. Typische Beispiele sind das (3R,6'R)-β-ε-Carotin-3,3'-diol (Lutein), (3R,3'S,5'R)-3,3'-Dihydroxy-β,κ-carotin-6-on (Capsanthin), das 9'-cis-6,6'-Diapocarotindisäure-6'-methylester (Bixin), (3S,3'S,5R,5'R)-3,3'-Dihydroxy-κ,κ-carotin-6,6'-dion (Capsorubin) oder das 3S,3'S)-3,3'-Dihydroxy-β,β'-carotin-4,4'-dion (Astaxanthin).

Neben den Carotinen und Carotinoiden sollen unter dem Begriff Carotinverbindungen auch deren Spaltprodukte wie beispielsweise 3,7-Dimethyl-9-(2,6,6-trimethyl-1-cyclohexenyl)-2,4,6,8-nonatetraen-1-ol (Retinol, Vitamin A1) und 3,7-Dimethyl-9-(2,6,6-trimethyl-1-cyclohexenyl)-2,4,6,8-nonatetraenal (Retinal, Vitamin A1-Aldehyd) verstanden werden. In einer bevorzugten Ausführungsform der vorliegenden Erfindung werden als Komponente (a) β-Carotin, Lutein oder deren Gemische im Gewichtsverhältnis 10 : 90 bis 90 : 10 und insbesondere 40 : 60 bis 60 : 40 eingesetzt.

### Tocopherole

Unter dem Begriff Tocopherole, die die Komponente (b) bilden, sind die in 2-Stellung mit einem 4,8,12-Trimethyltridecyl-Rest substituierten Chroman-6-ole (3,4-Dihydro-2H-1-benzopyran-6-ole) zu verstehen, die auch als Biochinone bezeichnet werden. Typische Beispiele sind die Plastichinone, Tocopherolchinone, Ubichinone, Bovichinone, K-Vitamine und Menachinone (z.B. 2-Methyl-1,4-naphthochinone). Vorzugsweise handelt es sich um die Chinone aus der Vitamin-E-Reihe, d.h. α-, β-, γ-, δ- und ε-Tocopherol, wobei letzteres noch über die ursprüngliche ungesättigte Prenylseitenkette verfügt (s. Abbildung).

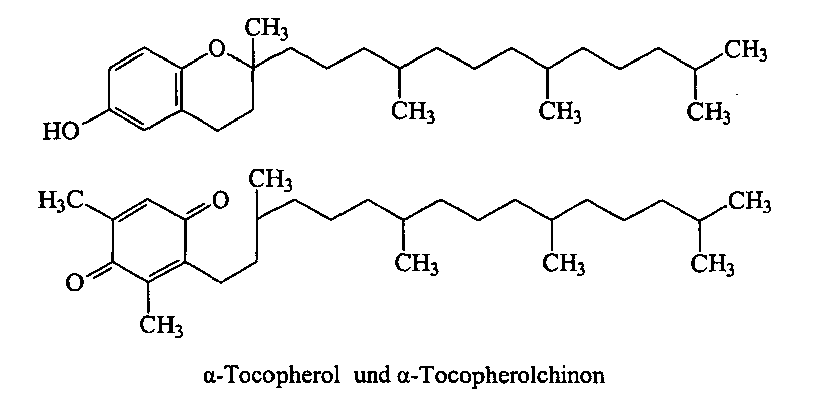

Daneben kommen auch Tocopherolchinone und -hydrochinone sowie die Ester der Chinone mit Carbonsäuren, wie z.B. Essigsäure oder Palmitinsäure in Frage. Der Einsatz von α-Tocopherol, Tocopherolacetat und Tocopherolpalmitat sowie deren Gemische ist bevorzugt.

### Extrakte der Passiflora incarnata

Früchte und Saaten der Pflanze *Passiflora incarnata*, also der Passionspflanze, deren Extrakte und aktive Prinzipien die Komponente (c) darstellen, sind reich an Flavonen des Typ Apigenin und Luteolin sowie deren C-Glykosiden :

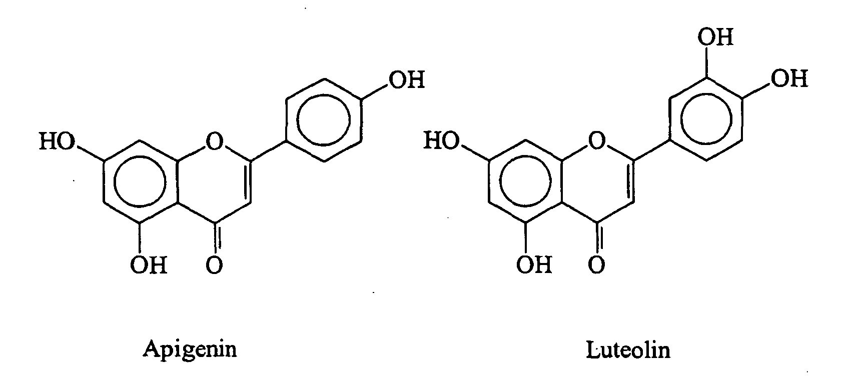

Darüber hinaus enthalten sie 2"-B-D-Glucoside, Schaftoside und Iso-Schaftoside, Vitexin, Isovitexin, Orientin, Isoorientin, Vicenin-2, Incenin-2, Saponanin sowie Spurenelemente, vor allem Calcium, Phosphor und Eisen.

### Extrakte der Vaccinium myrtillus

Früchte und Saaten der Pflanze *Vaccinium myrtillus*, also der gemeinen Heidel- oder Blaubeere, deren Extrakte und aktive Prinzipien die optionale Komponente (d) bilden, enthalten eine Mischung aus mindestens 15 verschiedenen Anthocyanosiden, wie beispielsweise Delphinidin:

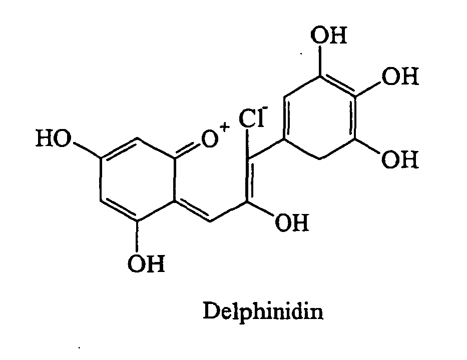

In der Regel enthalten die *Vaccinium*-Extrakte 20 bis 25 Gew.-% Anthocyanoside, 5 bis 10 Gew.-% Tannine sowie in geringen Mengen verschiedene Alkaloide (z.B. Myrtin und Epimyrtin), Phenolsäuren sowie Glycoside mit Quercitrin, Isoquercitrin und Hyperosid.

### Extraktion

Die Herstellung der Extrakte kann in an sich bekannter Weise erfolgen, d.h. beispielsweise durch wässrigen, alkoholischen oder wässrig-alkoholischen Auszug der Pflanzen bzw. Pflanzenteile bzw. der Blätter oder Früchte. Geeignet sind alle herkömmlichen Extraktionsverfahren wie z.B. Mazeration, Remazeration, Digestion, Bewegungsmazeration, Wirbelextraktion, Ultraschallextraktion, Gegenstromextraktion, Perkolation, Reperkolation, Evakolation (Extraktion unter vermindertem Druck), Diakolation oder Festflüssig-Extraktion unter kontinuierlichem Rückfluss. Für den großtechnischen Einsatz vorteilhaft ist die Perkolationsmethode. Als Ausgangsmaterial können frische Pflanzen oder Pflanzenteile eingesetzt werden, üblicherweise wird jedoch von getrockneten Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Gefriermahlung genannt. Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, verwendet werden. Besonders bevorzugt ist die Extraktion mit Methanol, Ethanol, Pentan, Hexan, Heptan, Aceton, Propylenglykolen, Polyethylenglykolen sowie Ethylacetat sowie Mischungen hieraus sowie deren wässrige Gemische. Die Extraktion erfolgt in der Regel bei 20 bis 100 °C, bevorzugt bei 30 bis 90 °C, insbesondere bei 60 bis 80 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem beliebigen Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Blätter liegen im Bereich von 3 bis 15, insbesondere 6 bis 10 Gew.-%. Die vorliegenden Erfindung umfasst die Erkenntnis, dass die Extraktionsbedingungen sowie die Ausbeuten der Endextrakte vom Fachmann ja nach gewünschtem Einsatzgebiet gewählt werden können. Diese Extrakte, die in der Regel Aktivsubstanzgehalte (= Feststoffgehalte) im Bereich von 0,5 bis 10 Gew.-% aufweisen, können als solche eingesetzt werden, es ist jedoch ebenfalls möglich, das Lösungsmittel durch Trocknung, insbesondere durch Sprüh- oder Gefriertrocknung vollständig zu entfernen, wobei ein intensiv rot gefärbter Feststoff zurückbleibt. Die Extrakte können auch als Ausgangsstoffe für die Gewinnung der oben genannten reinen Wirkstoffe dienen, sofern diese nicht auf synthetischem Wege einfacher und kostengünstiger hergestellt werden können. Demzufolge kann der Wirkstoffgehalt in den Extrakten 5 bis 100, vorzugsweise 50 bis 95 Gew.-% betragen. Die Extrakte selbst können als wässrige und/oder in organischen Solventien gelöste Zubereitungen sowie als sprüh- bzw. gefriergetrocknete, wasserfreie Feststoffe vorliegen. Als organische Lösungsmittel kommen in diesem Zusammenhang beispielsweise die aliphatischen Alkohole mit 1 bis 6 Kohlenstoffatomen (z.B. Ethanol), Ketone (z.B. Aceton), Halogenkohlenwasserstoffe (z.B. Chloroform oder Methylenchlorid), niedere Ester oder Polyole (z.B. Glycerin oder Glycole) in Frage.

### Zubereitungen für die orale oder topische Anwendung

In einer bevorzugten Ausführungsform der vorliegenden Erfindung können die Zubereitungen die Komponenten (a) bis (d) in folgenden Gewichtsverhältnissen
(a) 50 bis 90, vorzugsweise 70 bis 80 Gew.-% Carotinverbindungen,
(b) 5 bis 25, vorzugsweise 10 bis 20 Gew.-% Tocopherole,
(c) 5 bis 25, vorzugsweise 10 bis 15 Gew.-% Extrakte der *Passiflora incarnata* sowie
(d) 0 bis 10, vorzugsweise 1 bis 5 Gew.-% Extrakte der *Vaccinium myrtillus*
mit der Maßgabe enthalten, dass sich alle Mengenangaben auf den Wirkstoffgehalt beziehen und zu 100 Gew.-% ergänzen.

### Verkapselung

In einer besonderen Ausführungsform der vorliegenden Erfindung werden die oral bzw. topisch anzuwendenden Zubereitungen in verkapselter Form - beispielsweise in Gestalt üblicher Gelatinemakrokapseln - vorzugsweise aber in mikroverkapselter Form eingesetzt.

Unter den Begriffen "Mikrokapsel" oder "Nanokapsel" werden vom Fachmann sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Mikrokapseln und Verfahren zu ihrer Herstellung sind Gegenstand früherer Patenanmeldun**gen der Patentanmelderin [WO 01/01926, WO 01/01927, WO 01/01928, WO 01/01929].** Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
(a1) aus Gelbildnern, kationischen Polymeren und Wirkstoffen eine Matrix zubereitet,
(a2) gegebenenfalls die Matrix in einer Ölphase dispergiert,
(a3) die dispergierte Matrix mit wässrigen Lösungen anionischer Polymere behandelt und gegebenenfalls dabei die Ölphase entfernt.
oder
(b1) aus Gelbildnern, anionischen Polymeren und Wirkstoffen eine Matrix zubereitet,
(b2) gegebenenfalls die Matrix in einer Ölphase dispergiert,
(b3) die dispergierte Matrix mit wässrigen Kationpolymerlösungen behandelt und gegebenenfalls dabei die Ölphase entfernt;
oder
(c1) aus Gelbildnern und Wirkstoffen eine Matrix zubereitet,
(c2) die Matrix mit einer Kationpolymerlösung versetzt und
(c3) die Mischung auf einen pH-Wert einstellt der oberhalb des pKₛ-Wertes des Kationpolymers liegt;
oder
(d1) wässrige Wirkstoffzubereitungen mit Ölkörpern in Gegenwart von Emulgatoren zu O/W-Emulsionen verarbeitet,
(d2) die so erhaltenen Emulsionen mit wässrigen Lösungen anionischer Polymere behandelt,
(d3) die so erhaltene Matrix mit wässrigen Kationpolymerlösungen in Kontakt bringt und
(d4) die so erhaltenen Verkapselungsprodukte von der wässrigen Phase abtrennt;
oder
den Wirkstoff abwechselnd mit Schichten aus unterschiedlich geladenen Polyelektrolyten einhüllt (layer-by-layer-Technologie).

### • Gelbildner

Im Sinne der Erfindung werden als Gelbildner vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind.
Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

### • Kationische Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Vorzugsweise wird als Verkapselungsmaterial Chitosan eingesetzt. Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

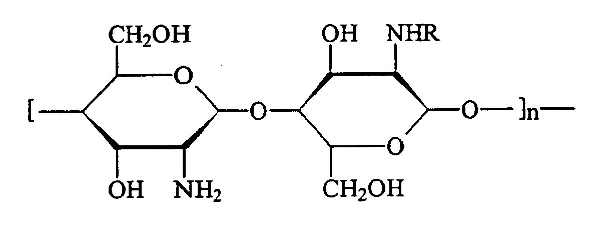

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

### • Ölphase

Die Matrix kann vor der Bildung der Membran optional in einer Ölphase dispergiert werden. Als Öle kommen für diesen Zweck beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglycerid-mischungen auf Basis von C₆-C₁₈₋Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂₋Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

### • Anionpolymere

Die anionische Polymere haben die Aufgabe, mit den Chitosanen Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein:

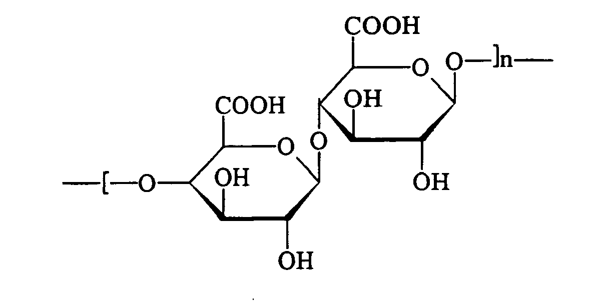

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.

### • Emulgatoren

Als Emulgatoren kommen anionische, amphotere, kationische oder vorzugsweise nichtionische oberflächenaktive Verbindungen aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole,
- Glycerincarbonat,
- aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure, sowie
- Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat.

### Herstellverfahren Mikrokapseln

Zur Herstellung der Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100°C, wird eine zweite wässrige Lösung zugegeben, welche das Kationpolymer, vorzugsweise das Chitosan in Mengen von 0,1 bis 2, vorzugsweise 0,25 bis 0,5 Gew.-% und den Wirkstoffen in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Kationpolymer und Wirkstoffen kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des Kationpolymers in der Matrix mit den anionischen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Anionpolymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 0,01 bis 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die Anionpolymere auch zur Herstellung der Matrix einsetzen und die Verkapselung mit den Kationpolymeren, speziell den Chitosanen durchführen.

Alternativ kann die Verkapselung auch unter ausschließlicher Verwendung von Kationpolymeren erfolgen, wobei man sich deren Eigenschaft zu Nutze macht, bei pH-Werten oberhalb des pKs-Wertes zu koagulieren.

In einem zweiten alternativen Verfahren wird zur Herstellung der erfindungsgemäßen Mikrokapseln wird zunächst eine O/W-Emulsion zubereitet, welche neben dem Ölkörper, Wasser und den Wirkstoffen eine wirksame Menge Emulgator enthält. Zur Herstellung der Matrix wird diese Zubereitung unter starkem Rühren mit einer entsprechenden Menge einer wässrigen Anionpolymerlösung versetzt. Die Membranbildung erfolgt durch Zugabe der Chitosanlösung. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diestern von Fettsäuren, Polyacrylaten, Polyacrylamiden und dergleichen noch unterstützt werden kann. Abschließend werden die Mikrokapseln von der wässrigen Phase beispielsweise durch Dekantieren, Filtrieren oder Zentrifugieren abgetrennt.

In einem dritten alternativen Verfahren erfolgt die Bildung der Mikrokapseln um einen vorzugsweise festen, beispielsweise kristallinen Kern, indem dieser schichtweise mit entgegengesetzt geladenen Polyelektrolyten eingehüllt wird. In diesem Zusammenhang sei auf das Europäische Patent **EP 1064088 B1** (Max-Planck Gesellschaft) verwiesen.

### Gewerbliche Anwendbarkeit

Die erfindungsgemäßen Zubereitungen zeichnen sich dadurch aus, dass sie über synergistische antioxidative und entzündungshemmende Eigenschaften verfügen und zusätzlich auch die Augen vor den schädlichen Einflüssen übermäßiger UV-Exposition schützen. Weitere Gegenstände der vorliegenden Erfindung betreffen daher die Verwendung der Mischungen als Nahrungsmittelergänzungsstoffe sowie zur Herstellung von Sonnenschutzmitteln, in denen sie in Mengen von 1 bis 10, vorzugsweise 2 bis 8 und insbesondere 3 bis 5 Gew.-% - bezogen auf die Endprodukte - enthalten sein können.

### Beispiele

### Beispiele 1 bis 4, Vergleichsbeispiele V1 bis V6

### Zellschutz gegenüber UV-A-Strahlen

Gegenstand der folgenden in-vitro Untersuchungen war festzustellen, ob die Testsubstanzen menschliche Fibroblasten vor oxidativem Stress, speziell der Einwirkung von UVA-Strahlen schützen können. UVA wurde deswegen als Stressfaktor gewählt, da die Strahlen bis in die Dermis eindringen und dort vor allem eine Lipoperoxdation der Cytoplasmamembranen bewirken. Die gebildeten Lipoperoxide zerfallen in Malonaldialdehyde (MDA), die für die Retikulation vieler Biomoleküle, wie z.B. Proteine (Enzyminhibierung) oder Nucleinbasen (Mutagenese) verantwortlich sind. Zur Versuchsdurchführung wurde eine Fibroblastenkultur mit fötalem Kalbsserum angesetzt und 2 Tage später mit jeweils 0,01 % w/v der Testsubstanzen geimpft. Nach einer Inkubation von 36 h bei 37 °C und einem CO₂-Level von 5 Vol.-% wurde das Nährmedium durch eine Elektrolytlösung ersetzt und die Fibroblasten mit einer definierten UVA-Strahlungsmenge geschädigt (3-15 J/cm²). Nach dem Ende der Bestrahlung wurde die Menge an gebildetem MDA in der überstehenden Lösung durch Umsetzung mit Thiobarbitursäure und der Gehalt an Proteinen im Zellhomogenisat nach der Bradford-Methode bestimmt. Die Ergebnisse sind in Tabelle 1 zusammengefasst. Die Mengenangaben verstehen sich als Gew.-%, die Angaben zum Wirkungsnachweis als %-rel gegenüber dem Standard, d.h. Bestrahlung ohne Zugabe von Testsubstanzen. Angegeben ist der Mittelwert von zwei Messreihen mit Dreifachbestimmung. Die Beispiele 1 bis 4 sind erfindungsgemäß, die Beispiele V1 bis V6 dienen zum Vergleich.

**Tabelle 1**

| **Wirkung gegen UV-A-Strahlen** | | | | | | | | | | |
|---|---|---|---|---|---|---|---|---|---|---|
| **Komponente** | **V1** | **V2** | **V3** | **V4** | **V5** | **V6** | **1** | **2** | **3** | **4** |
| Beta-Karotin | - | 100 | - | - | - | 50 | 75 | - | 60 | 60 |
| Lutein | - | - | 100 | - | - | - | - | 75 | 15 | 15 |
| Tocopherolacetat | - | - | - | 100 | - | 50 | 5 | 5 | 5 | 5 |
| Passiflora-Extrakt | - | - | - | - | 100 | - | 15 | 15 | 15 | 10 |
| Vaccinium-Extrakt | - | - | - | - | - | - | - | - | - | 5 |
| ***Wirkungsnachweis*** | | | | | | | | | | |
| Freigesetztes MDA | 100 | 80 | 78 | 84 | 100 | 73 | 62 | 65 | 60 | 58 |
| Cellulare Proteine | 100 | 84 | 76 | 82 | 99 | 75 | 70 | 68 | 58 | 60 |

### Beispiele 5 bis 8, Vergleichsbeispiele V7 bis V12

### Zellschutz gegenüber UV-B-Strahlen

Aufgabe dieses Tests war es zu zeigen, dass die Testsubstanzen anti-inflammatorische Eigenschaften gegenüber menschlichen Keratinocyten besitzen. UVB wurde als Stressfaktor ausgewählt, weil die Strahlen durch Aktivierung von Arachidonsäure freisetzenden Enzymen, wie beispielsweise Phospholipase A2 (PLA2) eine cutane Inflammation (Erytheme, Ödeme) hervorrufen. Dies führt in der Folge nicht nur zu einer Schädigung der Membranen, sondern auch zur Bildung von inflammatorisch wirkenden Stoffen, wie beispielsweise Prostaglandinen vom Typ PGE2. Der Einfluss der UVB-Strahlen auf die Keratinocyten wurde in-vitro über die Freisetzung von cytoplasmatischen Enzymen, wie z.B. LDH (Lactat Dehydrogenase) bestimmt, die parallel zur Zellschädigung und der Bildung von PGE2 verläuft. Zur Versuchsdurchführung wurde eine Fibroblastenkultur mit fötalem Kalbsserum angesetzt und 2 Tage später mit jeweils 0,01 % w/v der Testsubstanzen geimpft. Nach einer Inkubation von 36 h bei 37 °C und einem CO₂-Level von 5 Vol.-% wurde das Nährmedium durch eine Elektrolytlösung ersetzt und die Fibroblasten mit einer definierten UVB-Strahlungsmenge geschädigt (50 mJ/cm²). Die Keratinocytenmenge wurde nach Trypsination über einen Zellzähler ermittelt, die LDH-Konzentration enzymatisch bestimmt. Die Ergebnisse sind in Tabelle 2 zusammengefasst. Angegeben ist die Aktivität in %-rel gegen einen Standard, d.h. Bestrahlung ohne Zugabe von Testsubstanzen als Mittelwert von zwei Testreihen mit Doppelbestimmung. Die Beispiele 5 bis 8 sind erfindungsgemäß, die Beispiele V7 bis V12 dienen zum Vergleich.

**Tabelle 2**

| **Wirkung gegen UVB-Strahlen** | | | | | | | | | | |
|---|---|---|---|---|---|---|---|---|---|---|
| **Komponente** | **V7** | **V8** | **V9** | **V10** | **V11** | **V12** | **5** | **6** | **7** | **8** |
| Beta-Karotin | - | 100 | - | - | - | 50 | 75 | - | 60 | 60 |
| Lutein | - | - | 100 | - | - | - | - | 75 | 15 | 15 |
| α-Tocopherol | - | - | - | 100 | - | 50 | 5 | 5 | 5 | 5 |
| *Passiflora-*Extrakt | - | - | - | - | 100 | - | 15 | 15 | 15 | 10 |
| *Vaccinium-Extrakt* | - | - | - | - | - | - | - | - | - | 5 |
| ***Wirkungsnachweis*** | | | | | | | | | | |
| Cellulare DNA | 31 | 18 | 18 | 22 | 30 | 17 | 10 | 8 | 5 | 5 |
| Freigesetztes LDH | 100 | 84 | 80 | 76 | 98 | 79 | 66 | 63 | 59 | 57 |
| Freigesetztes PGE2 | 100 | 86 | 79 | 78 | 100 | 81 | 66 | 59 | 55 | 55 |

### Beispiel 9

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer Lösung von 10 g Glycerin 90 ml Wasser und dann mit einer Zubereitung von 2,5 g Natriumalginat in Form einer 10 Gew.-%igen wässrigen Lösung, 3 g Beta-Carotin (Betatene® , Cognis), 1 g α-Tocopherol (Covitox® , Cognis), 0,2 g getrockneter *Passiflora incarnata* Extrakt (Herbalia® Passiflora, Cognis 0,2 g getrockneter *Vaccinium myrtillus* Extrakt (Herbalia® Bilberry, Cognis), 0,5 g Phenonip® und 0,5 g Polysorbat-20 (Tween® 20, ICI) in 64 g Wasser versetzt. Die erhaltene Matrix wurde filtriert, auf 60 °C erwärmt und in eine 1 Gew.-%ige Lösung von Chitosanglycolat in Wasser getropft. Zum Erhalt von Mikrokapseln gleichen Durchmessers wurden die Zubereitungen anschließend gesiebt.

### Beispiel 10

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer Lösung von 10 g Glycerin 90 ml Wasser und dann mit einer Zubereitung von 2,5 g Natriumalginat in Form einer 10 Gew.-%igen wässrigen Lösung, 1 g Beta-Carotin (Betatene® , Cognis), 1 g Lutein, 1 g α-Tocopherol (Covitox® , Cognis), 0,2 g getrockneter *Passiflora incarnata* Extrakt (Herbalia® Passiflora, Cognis), 0,2 g getrockneter *Vaccinium myrtillus* Extrakt (Herbalia® Bilberry, Cognis), 0,5 g Phenonip® und 0,5 g Polysorbat-20 (Tween® 20, ICI) in 64 g Wasser versetzt. Die erhaltene Matrix wurde filtriert, auf 60 °C erwärmt und in eine 1 Gew.-%ige Lösung von Chitosanglycolat in Wasser getropft. Zum Erhalt von Mikrokapseln gleichen Durchmessers wurden die Zubereitungen anschließend gesiebt.

In der nachfolgenden Tabelle 3 finden sich eine Reihe von Rezepturen für topisch anzuwendende Sonnenschutzmittel.

**Tabelle 3**

| **Sonnenschutzmittel (Wasser, Konservierungsmittel ad 100 Gew.-%)** | | | | | | | | | | |
|---|---|---|---|---|---|---|---|---|---|---|
| **Zusammensetzung (INCI)** | **1** | **2** | **3** | **4** | **5** | **6** | **7** | **8** | **9** | **10** |
| **Dehymuls® PGPH** | 4,0 | 3,0 | - | 5,0 | - | - | - | - | - | - |
| Polyglyceryl-2 Dipolyhydroxystearate | | | | | | | | | | |
| **Lameform® TGI** | 2,0 | 1,0 | - | - | - | - | - | - | - | - |
| Polyglyceryl-3 Diisostearate | | | | | | | | | | |
| **Emulgade® PL 68/50** | - | - | - | - | 4,0 | - | - | - | 3,0 | - |
| Cetearyl Glucoside (and) Cetearyl Alcohol | | | | | | | | | | |
| **Eumulgin®B2** | - | - | - | - | - | - | - | 2,0 | - | - |
| Ceteareth-20 | | | | | | | | | | |
| **Tegocare®** PS | - | - | 3,0 | - | - | - | 4,0 | - | - | - |
| Polyglyceryl-3 Methylglucose Distearate | | | | | | | | | | |
| **Eumulgin VL 75** | - | - | - | - | - | 3,5 | - | - | 2,5 | - |
| Polyglyceryl-2 Dipolyhydroxystearate (and) Lauryl Glucoside (and) Glycerin | | | | | | | | | | |
| **Bees Wax** | 3,0 | 2,0 | 5,0 | 2,0 | - | - | - | - | - | - |
| **Cutina® GMS** | - | - | - | - | - | 2,0 | 4,0 | - | - | 4,0 |
| Glyceryl Stearate | | | | | | | | | | |
| **Lanette® O** | - | - | 2,0 | - | 2,0 | 4,0 | 2,0 | 4,0 | 4,0 | 1,0 |
| Cetearyl Alcohol | | | | | | | | | | |
| **Antaron® V 216** | - | - | - | - | - | 3,0 | - | - | - | 2,0 |
| PVP / Hexadecene Copolymer | | | | | | | | | | |
| **Myritol® 818** | 5,0 | - | 10,0 | - | 8,0 | 6,0 | 6,0 | - | 5,0 | 5,0 |
| Cocoglycerides | | | | | | | | | | |
| **Finsolv® TN** | - | 6,0 | - | 2,0 | - | - | 3,0 | - | - | 2,0 |
| C12/15 Alkyl Benzoate | | | | | | | | | | |
| **Cetiol® J 600** | 7,0 | 4,0 | 3,0 | 5,0 | 4,0 | 3,0 | 3,0 | - | 5,0 | 4,0 |
| Oleyl Erucate | | | | | | | | | | |
| **Cetiol® OE** | 3,0 | - | 6,0 | 8,0 | 6,0 | 5,0 | 4,0 | 3,0 | 4,0 | 6,0 |
| Dicaprylyl Ether | | | | | | | | | | |
| **Mineral Oil** | - | 4,0 | - | 4,0 | - | 2,0 | - | 1,0 | - | - |
| **Cetiol® PGL** | - | 7,0 | 3,0 | 7,0 | 4,0 | - | - | - | 1,0 | - |
| Hexadecanol (and) Hexyldecyl Laurate | | | | | | | | | | |
| **Bisabolol** | 1,2 | 1,2 | 1,2 | 1,2 | 1,2 | 1,2 | 1,2 | 1,2 | 1,2 | 1,2 |
| **Mikrokapseln gemäß Bsp. 10** | 1,0 | 1,0 | 1,0 | 1,0 | 1,0 | 1,0 | 1,0 | 1,0 | 1,0 | 1,0 |
| **Hydagen® CMF** | 1,0 | 1,0 | 1,0 | 1,0 | 1,0 | 1,0 | 1,0 | 1,0 | 1,0 | 1,0 |
| Chitosan | | | | | | | | | | |
| **Copberol® F 1300** | 0,5 | 1,0 | 1,0 | 2,0 | 1,0 | 1,0 | 1,0 | 2,0 | 0,5 | 2,0 |
| Tocopherol / Tocopheyl Acetate | | | | | | | | | | |
| Neo **Heliopan® Hydro** | 3,0 | - | - | 3,0 | - | - | 2,0 | - | 2,0 | - |
| Sodium Phenylbenzimidazole Sulfonate | | | | | | | | | | |
| Neo **Heliopan® 303** | - | 5,0 | - | - | - | 4,0 | 5,0 | - | - | 10,0 |
| Octocrylene | | | | | | | | | | |
| **Neo Heliopan® BB** | 1,5 | - | - | 2,0 | 1,5 | - | - | - | 2,0 | - |
| Benzophenone-3 | | | | | | | | | | |
| **Neo Heliopan® E 1000** | 5,0 | - | 4,0 | - | 2,0 | 2,0 | 4,0 | 10,0 | - | - |
| Isoamyl p-Methoxycinnamate | | | | | | | | | | |
| **Neo Heliopan® AV** | 4,0 | - | 4,0 | 3,0 | 2,0 | 3,0 | 4,0 | - | 10,0 | 2,0 |
| Octyl Methoxycinnamate | | | | | | | | | | |
| **Uvinul® T 150** | 2,0 | 4,0 | 3,0 | 1,0 | 1,0 | 1,0 | 4,0 | 3,0 | 3,0 | 3,0 |
| Octyl Triazone | | | | | | | | | | |
| **Zinc Oxide** | - | 6,0 | 6,0 | - | 4,0 | - | - | - | - | 5,0 |
| **Titanium Dioxide** | - | - | - | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |

| | | | | | | | | | | |
|---|---|---|---|---|---|---|---|---|---|---|
| (1) W/O-Sonnenschutzcreme, (2-4) W/O-Sonnenschutzlotion, (5, 8, 10) O/W-Sonnenschutzlotion (6, 7, 9) O/W-Sonnenschutzcreme | | | | | | | | | | |

## Patentansprüche

1. Zubereitungen für die orale und/oder topische Anwendung, enthaltend
(a) Carotinverbindungen,
(b) Tocopherole und
(c) Extrakte der Pflanze *Passiflora incarnata* bzw. deren aktive Wirkstoffe sowie gegebenenfalls
(d) Extrakte der Pflanze *Vaccinium myrtillus* bzw. deren aktive Wirkstoffe.

2. Zubereitungen nach Anspruch 1, **dadurch gekennzeichnet, dass** sie als Komponente (a) β-Carotin, Lutein oder deren Gemische enthalten.

3. Zubereitungen nach den Ansprüchen 1 und/oder 2, **dadurch gekennzeichnet, dass** sie als Komponente (b) α-Tocopherol, Tocopherolacetat und Tocopherolpalmitat oder deren Gemische enthalten.

4. Zubereitungen nach mindestens einem der Ansprüche 1 bis 3, **dadurch gekennzeichnet, dass** sie als aktive Wirkstoffe der *Passiflora incarnata* (Komponente c) Flavone vom Typ Apigenin, Luteolin oder deren C-Glycoside sowie Vitexin und Isovitexin enthalten.

5. Zubereitungen nach mindestens einem der Ansprüche 1 bis 4, **dadurch gekennzeichnet, dass** sie als aktive Wirkstoffe der *Vaccinium myrtillus* (optionale Komponente d) Anthocyanoside vom Typ Delphinidin enthalten.

6. Zubereitungen nach mindestens einem der Ansprüche 1 bis 5, **dadurch gekennzeichnet, dass** sie
(a) 50 bis 90 Gew.-% Carotinverbindungen,
(b) 5 bis 25 Gew.-% Tocopherole,
(c) 5 bis 25 Gew.-% Extrakte der *Passiflora incarnata* sowie
(d) 0 bis 10 Gew.-% Extrakte der *Vaccinium myrtillus*
mit der Maßgabe enthalten, dass sich alle Mengenangaben auf den Wirkstoffgehalt beziehen und zu 100 Gew.-% ergänzen.

7. Zubereitungen nach mindestens einem der Ansprüche 1 bis 6, **dadurch gekennzeichnet, dass** sie in verkapselter Form vorliegen.

8. Zubereitungen nach Anspruch 7, **dadurch gekennzeichnet, dass** sie als Gelatinemakrokapseln vorliegen.

9. Verwendung von Zubereitungen nach Anspruch 1 zur Herstellung von Nahrungsmittelergänzungsstoffen.

10. Verwendung von Zubereitungen nach Anspruch 1 zur Herstellung von Sonnenschutzmitteln.

## Claims

1. Preparations for oral and/or topical application containing
(a) carotene compounds,
(b) tocopherols and
(c) extracts of the plant *Passiflora incarnata* or active components thereof and optionally
(d) extracts of the plant *Vaccinium myrtillus* or active components thereof.

2. Preparations as claimed in claim 1, **characterized in that** they contain β-carotene, lutein or mixtures thereof as component (a).

3. Preparations as claimed in claims 1 and/or 2, **characterized in that** they contain α-tocopherol, tocopherol acetate and tocopherol palmitate or mixtures thereof as component (b).

4. Preparations as claimed in at least one of claims 1 to 3, **characterized in that** they contain flavones such as apigenin, luteolin or C-glycosides thereof and vitexin and isovitexin as active components of *Passiflora incarnata* (component c).

5. Preparations as claimed in at least one of claims 1 to 4, **characterized in that** they contain anthocyanosides of the delphinidin type as active components of *Vaccinium myrtillus* (optional component d).

6. Preparations as claimed in at least one of claims 1 to 5, **characterized in that** they contain
(a) 50 to 90% by weight carotene compounds,
(b) 5 to 25% by weight tocopherols,
(c) 5 to 25% by weight extracts of *Passiflora incarnata* and
(d) 0 to 10% by weight extracts of *Vaccinium myrtillus,*
with the proviso that the quantities shown are based on the active substance content and add up to 100% by weight

7. Preparations as claimed in at least one of claims 1 to 6, **characterized in that** they are present in encapsulated form.

8. Preparations as claimed in claim 7, **characterized in that** they are present as gelatine microcapsules.

9. Use of the preparations claimed in claim 1 for the production of food supplements.

10. Use of the preparations claimed in claim 1 for the production of sun protection agents.

## Revendications

1. Préparations pour application orale et/ou topique, comprenant :
(a) des composés de carotène,
(b) des tocophérols et
(c) des extraits végétaux de *Passiflora incarnata* ou ses principes actifs ainsi qu'éventuellement
(d) des extraits végétaux de *Vaccinium myrtillus* ou ses principes actifs.

2. Préparations selon la revendication 1,
**caractérisées en ce que**
comme composant (a) elles contiennent de la β-carotène, de la lutéine ou des mélanges de celles-ci.

3. Préparations selon les revendications 1 et/ou 2,
**caractérisées en ce que**
comme composant (b) elles contiennent de l'α-tocophérol, de l'acétate de tocophérol et du palmitate de tocophérol ou des mélanges de ceux-ci.

4. Préparations selon au moins l'une des revendications 1 à 3,
**caractérisées en ce que**
comme principes actifs de *Passiflora incarnata* (composant c) elles contiennent des flavones du type apigénine, lutéoline ou leurs C-glycosides ainsi que de la vitexine et de l'isovitexine.

5. Préparations selon au moins l'une des revendications 1 à 4,
**caractérisées en ce que**
comme principes actifs de *Vaccinium myrtillus* (composant d facultatif) elles contiennent des anthocyanosides du type delphinidine.

6. Préparations selon au moins l'une des revendications 1 à 5,
**caractérisées en ce qu'**
elles contiennent
(a) de 50 à 90 % en poids de composés de carotène,
(b) de 5 à 25 % en poids de tocophérols,
(c) de 5 à 25 % en poids d'extraits de Passiflora incarnata ainsi que
(d) de 0 à 10 % en poids d'extraits de Vaccinium myrtillus,
étant précisé que toutes les indications quantitatives sont rapportées à la teneur en principe actif et se complètent pour faire 100 % en poids.

7. Préparations selon au moins l'une des revendications 1 à 6,
**caractérisées en ce qu'**
elles se présentent sous une forme encapsulée.

8. Préparations selon la revendication 7,
**caractérisées en ce qu'**
elles se présentent sous la forme de macrocapsules en gélatine.

9. Utilisation des préparations selon la revendication 1, pour l'obtention de compléments alimentaires.

10. Utilisation des préparations selon la revendication 1, pour l'obtention de produits de protection solaire.
